# EUROPEAN PATENT SPECIFICATION

(11) **EP 2 832 284 B1**
(45) Date of publication and mention of the grant of the patent: **18.07.2018**
(21) Application number: 13770001.9
(22) Date of filing: 28.03.2013
(51) Int. Cl.: A61B 1/00

(54) **INTRACORPOREAL INTRODUCTION DEVICE**
INTRAKORPORALE EINFÜHRUNGSVORRICHTUNG
DISPOSITIF D'INTRODUCTION INTRACORPORELLE

(30) Priority: 30.03.2012 JP 2012081812
(43) Date of publication of application: 04.02.2015
(73) Proprietor: Olympus Corporation, Tokyo 192-8507 (JP)
(72) Inventor: ISHIZAKI, Ryosuke, Hachioji-shi, Tokyo 192-8507 (JP); NISHIIE, Takehiro, Hachioji-shi, Tokyo 192-8507 (JP)
(74) Representative: Gunzelmann, Rainer
(86) International application number: PCT/JP2013/059248
(87) International publication number: WO 2013/147017

(56) References cited:
- WO-A2-2006/125187
- JP-A- H1 128 249
- JP-A- 2008 284 161
- US-A1- 2012 029 281

## Description

### BACKGROUND OF THE INVENTION

### 1. Field of the Invention

The present invention relates to a living body introduction apparatus provided on a leading end side of an insertion portion.

### 2. Description of the Related Art

Endoscope apparatuses are generally known which comprise an insertion section inserted into a lumen to allow observation of images picked up using an image pickup section provided at a leading end portion of the insertion section. For example, U.S. Patent Application No. US2012/0029281A1 proposes an endoscope apparatus with a living body introduction apparatus which is placed after a leading end of an insertion section or a bending section at the leading end is placed and which rotates around a longitudinal axis.

The living body introduction apparatus comprises a fin provided on a surface of an outer rotating cylinder of the insertion section and comprising a soft material such as rubber or resin, the fin being formed to wrap spirally around the surface. The fin is rotated while being inserted through the lumen to perform an auxiliary operation for insertion of the insertion section by advancing while pushing the inner wall, fold, and the like of the lumen away backward.

The living body introduction apparatus draws a rotational driving force exerted by a motor provided in a manipulation section of the endoscope and serving as a driving source, into the insertion section through a wire or the like to rotate a driving gear provided at a leading end of a drive shaft. The gear allows an inner rotating cylinder to be fitted over an outer circumference of the insertion section. The rotating cylinder comprises gear teeth annularly provided along an inner circumferential surface of the rotating cylinder. The rotating cylinder fit on the outer circumferential surface of the insertion section.

Moreover, plurality of rollers is arranged on an upper surface of the inner rotating cylinder so as to be rotatable in position in a longitudinal direction of the insertion section. The rollers are arranged in alignment with recesses (roller spacings) formed by a pair of small rollers provided on an inner surface side of the outer rotating cylinder, to transmit a rotating force of the inner rotating cylinder to the outer rotating cylinder. Thus, the outer rotating cylinder rotates integrally with the inner rotating cylinder in conjunction with rotation of the inner rotating cylinder.

The above-described living body introduction apparatus is inserted into the lumen in a living body or the like for use and thus needs to have a liquid-tight structure. That is, the liquid-tight structure is needed in order to prevent structural sites from being corroded as a result of inward infiltration of a liquid such as a body fluid and to ensure a sufficient sterilization effect.

In a rotating mechanism comprising the inner rotating cylinder and the outer rotating cylinder and utilizing the plurality of rollers, the inner rotating cylinder including the rollers is covered with a cover comprising a tube-like waterproof sheet. The cover allows openings at opposite ends of the cover to be fixed in a liquid-tight manner to non-rotating insertion sections (longitudinal axis direction sides of the insertion section) that support the inner rotating cylinder so that the inner rotating cylinder is rotatable. This configuration allows the rollers to rotate on the inner rotating cylinder while pushing the cover up so that the cover forms protruding portions when the inner rotating cylinder rotates inside the cover. Each of the protruding portions is fitted into the recess between the small rollers pairs spaced from one another on the inner surface side of the outer rotating cylinder. Thus, pushing the small roller pairs ahead rotates the outer rotating cylinder.

### BRIEF SUMMARY OF THE INVENTION

The invention is defined in independent claim 1. Further aspects and preferred embodiments are defined in the appended claims. Aspects, embodiments and examples of the present disclosure which do not fall under the scope of the appended claims do not form part of the invention and are merely provided for illustrative purpose. Advantages of the invention will be set forth in the description which follows, and in part will be obvious from the description, or may be learned by practice of the invention. The advantages of the invention may be realized and obtained by means of the instrumentalities and combinations particularly pointed out hereinafter.

### BRIEF DESCRIPTION OF THE SEVERAL VIEWS OF THE DRAWINGS

The accompanying drawings, which are incorporated in and constitute a part of the specification, illustrate embodiments of the invention, and together with the general description given above and the detailed description of the embodiments given below, serve to explain the principles of the invention.
FIG. 1 is a conceptual diagram showing an external configuration of an endoscope apparatus with a living body introduction apparatus on an insertion section according to a first embodiment.
FIG. 2 is a diagram showing a configuration of the living body introduction apparatus in section.
FIG. 3A is a cross-sectional view showing the configuration and taken along A-A in FIG. 2.
FIG. 3B is a simplified cross-sectional view taken along A-A in FIG. 2.
FIG. 4A is a diagram illustrating an assembly structure of the living body introduction apparatus.
FIG. 4B is a diagram continued from FIG. 4A and illustrating the assembly structure of the living body introduction apparatus.
FIG. 4C is a diagram continued from FIG. 4B and illustrating the assembly structure of the living body introduction apparatus.
FIG. 4D is a diagram continued from FIG. 4C and illustrating the assembly structure of the living body introduction apparatus.
FIG. 5A is a diagram continued from FIG. 4D and illustrating the assembly structure of the living body introduction apparatus.
FIG. 5B is a diagram continued from FIG. 5A and illustrating the assembly structure of the living body introduction apparatus.
FIG. 5C is a diagram continued from FIG. 5B and illustrating the assembly structure of the living body introduction apparatus.
FIG. 5D is a diagram continued from FIG. 5C and illustrating the assembly structure of the living body introduction apparatus.
FIG. 6 is a diagram showing a configuration of a living body introduction apparatus according to a second embodiment in section0.
FIG. 7 is a cross-sectional view showing the configuration and taken along B-B in FIG. 6.
FIG. 8 is a diagram showing an external configuration of an outer rotating cylinder configured integrally with a spiral tube (a casing) of the living body introduction apparatus.
FIG. 9A is a diagram showing, in section, the configuration in which a cover is slackened or creased.

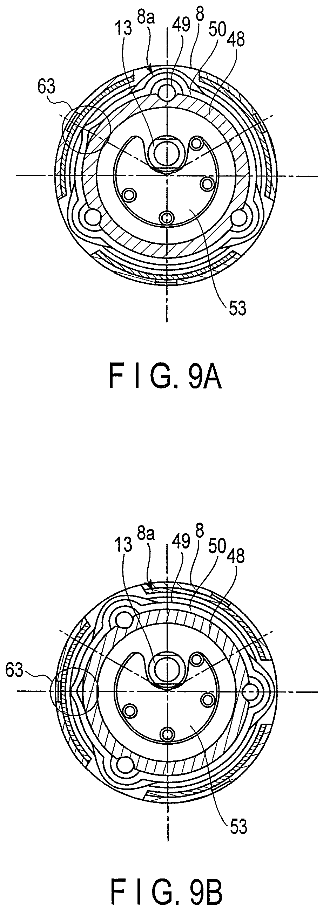

FIG. 9A is a diagram showing, in section, the configuration in which the cover is slackened or creased.

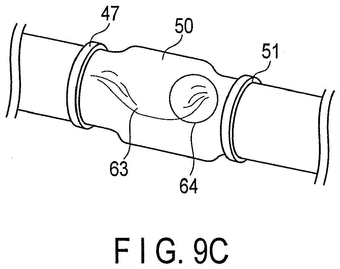

FIG. 9C is a diagram showing the sideways appearance of the outer rotating cylinder with the cover slackened or creased.

### DETAILED DESCRIPTION OF THE PREFERRED EMBODIMENTS

Embodiments of the present invention will be described below in detail with reference to the drawings.

### [Embodiment 1]

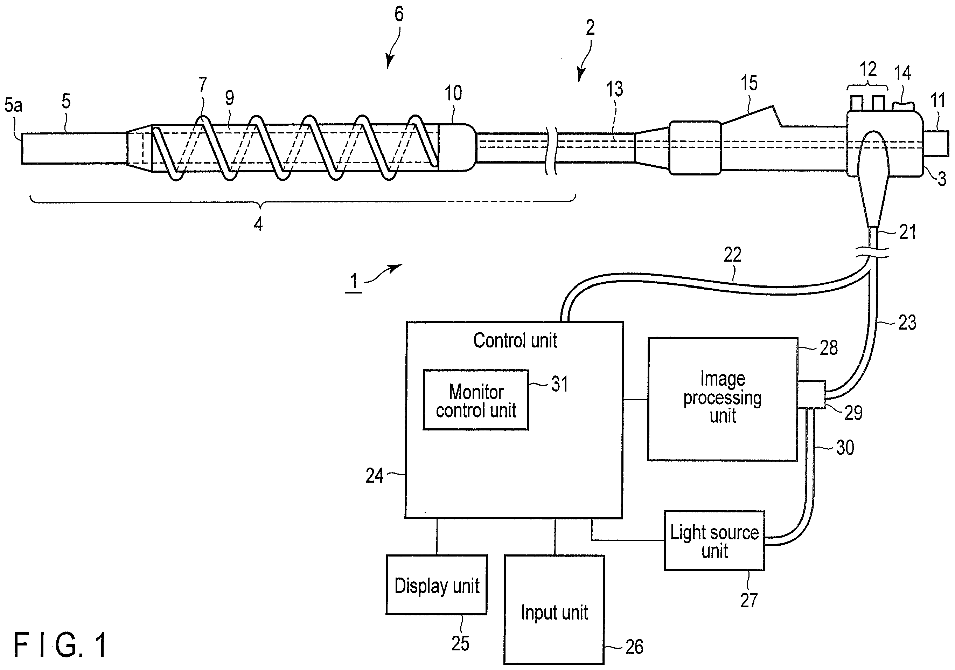

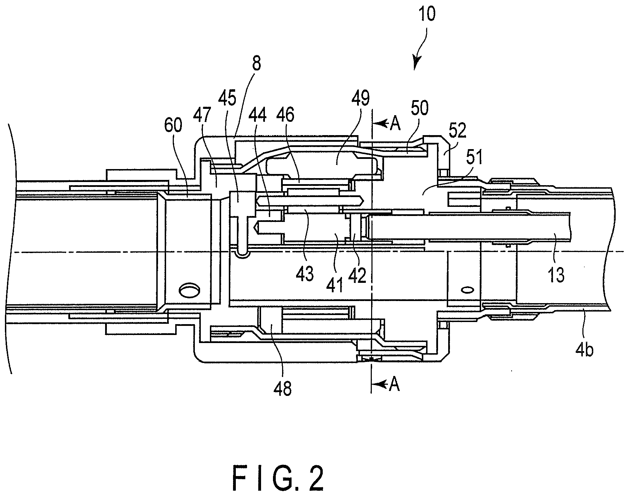

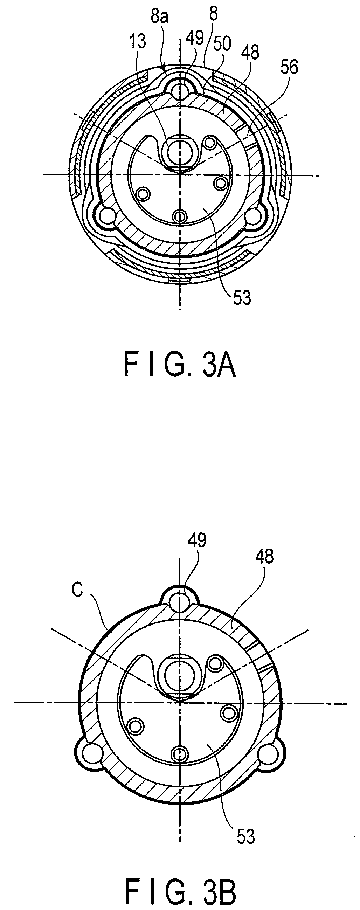

FIG. 1 is a conceptual diagram showing an external configuration of an endoscope apparatus with an insertion section on which a living body introduction apparatus according to a first embodiment is provided. FIG. 2 is a diagram showing a configuration of the living body introduction apparatus in section. FIG. 3A is a cross-sectional view showing the configuration and taken along A-A in FIG. 2.

An endoscope apparatus 1 according to the present embodiment comprises an insertion section 2, a manipulation section 3 provided on a base end side of the insertion section 2, and a driving unit system.

The insertion section 2 comprises a bending section 5 formed to conform with bending of a lumen. The bending section 5 comprises an image pickup section, a wash nozzle, and a channel hole all formed at a leading end portion 5a of the bending section 5; a pair of forceps or the like is inserted through the channel hole. A living body introduction apparatus 6 described below is provided at a position backward of the leading end of the insertion section 2 at any distance from the leading end. A member insertion section 15 with an opening end of the channel formed therein is disposed on a base end side of the insertion section 2 and between the insertion section 2 and the manipulation section 3.

A knob and a switch 12 allowing various manipulations including a bending manipulation to be performed are disposed on the manipulation section 3. A motor 11 described below is disposed opposite the base end side of the insertion section 2, and an operation switch 14 for driving the motor is disposed in the vicinity of the motor 11. The motor 11 may be contained in the main body of the manipulation section 3.

The driving unit system comprises a control section 24 that controls the whole apparatus including units, an image processing unit 28 that processes images picked up using the image pickup section, a light source unit 27 that emits illumination light illuminating an image pickup target, an input unit 26 that inputs instructions and the like to the control section 24, and a display unit 25 that displays picked-up images. In the description below, electric or optical connections using connectors are referred to as connector connections.

The image processing unit 28 is connector-connected to the manipulation section 3 through a universal cable 23 (integrated cable 21) via a scope connector 29. The light source unit 27 is coupled to the universal cable 23 through a light guide cable 30 via the scope connector 29. The universal cable 23 is configured to include at least a video signal cable through which video signals obtained using the image pickup section are communicated to the image processing unit and a cable (optical fiber) through which illumination light is guided. According to the present embodiment, the universal cable 23 and a control cable 22 extending from the control section 24 are connected to the manipulation section as the integrated cable 3 by being combined together before reaching the manipulation section 3. Of course, the integrated connector connection need not necessarily be used but the units are connected to the manipulation section 3 through the respective cables.

The display unit 25 is configured using, for example, a liquid crystal monitor and can also display a picked-up image and information (for example, position information) on the image in the same screen. The control section 24 includes a motor control section 31 that controllably drives the motor 11. The control section 24 is not limited to a dedicated apparatus but may be configured utilizing a general-purpose processing apparatus, for example, as a personal computer with programs mounted therein.

The insertion section 2 comprises an insertion section main body 4 extending along a longitudinal direction of the insertion section 2, and a bending section 5 provided on a leading end side of the insertion section 2. The insertion section 2 also comprises a rotational driving section 10 provided on the insertion section main body 4 to enable a spiral tube (an introduction propulsion section) 9 to rotate around the longitudinal direction of the insertion section 2, the spiral tube 9 rotating around a longitudinal axis of the insertion section 2 to function as introduction propulsion. In other words, the insertion section main body 4 is formed by connecting the bending section 5, a front flexible hollow shaft 4a, the rotational driving section 10, and a rear flexible hollow shaft 4b together in this order from the leading end side of the insertion section main body 4. A base end portion of the bending section 5 is connected to a front ferrule of the front flexible hollow shaft 4a. A rear ferrule of the front flexible hollow shaft 4a is fitted in and connected to a flange portion of a front cylinder 47 of the rotational driving section 10 as shown in FIG. 2. A front ferrule of the rear flexible hollow shaft 4b is fitted in and connected to an inner circumference of the flange portion of a gear cylinder 51 of the rotational driving section 10. The bending section 5 has a well-known configuration and comprises a plurality of joint rings (not shown in the figure) coupled together so as to be rotationally movable alternately using a lateral direction and a vertical direction as a supporting point. One end of a bending wire (not shown in the figure) is fixed to each of the joint rings. The other end of each bending wire is connected to the knob (not shown in the figure) provided on the manipulation section 3. Operation of the knob allows the bending wires to be pulled in the vertical direction and the lateral direction to tilt and bend the joint rings, to which the bending wires are coupled.

Moreover, a living body introduction apparatus 6 is provided on the insertion section 2.

The living body introduction apparatus 6 comprises a fin 7 formed so as to wrap spirally around the living body introduction apparatus 6 and functioning as a propulsion site (or a backward site). The living body introduction apparatus 6 also comprises the spiral tube (the introduction propulsion section) 9 provided over a casing of the insertion section 2 with a void between the casing and the spiral tube 9 and rotating around the longitudinal axis of the insertion section 2 to function as introduction propulsion. A leading end side of the spiral tube 9 is tapered and thus shaped so as to be easily inserted. The spiral tube 9 can be rotated in both directions (CW and CCW) by the rotational driving section 10. The motor 11, serving as a driving source for the rotational driving section 10, is disposed on the manipulation section 3. One end of a flexible bending shaft 13 is coupled to a rotating shaft of the rotational driving section 10. Furthermore, the shaft 13 penetrates the insertion section 2 and is coupled to one end of a shaft of a driving gear 41 at the other end of the shaft 13 using a coupling member 42.

A rotating force of the motor 11 is transmitted through the flexible bending shaft 13 to rotate the driving gear 41. The coupling member 42 may be configured to allow, during transmission of motor rotation, intervention of a mechanism comprising a plurality of gears for controlling a decrease in the number of rotations and adjusting torque.

The rotating force of the motor 11 is utilized to rotate the spiral tube 9 and rotates the spiral tube 9 in a direction opposite to a spiral direction in which the fin wraps around the living body introduction apparatus to advance the insertion section 2. The spiral tube 9 is removable fitted over the insertion section 2 and comprises a material (for example, a rubber material or a resin material), a structure, or the like which is flexible enough to conform with bending of the bending section 5.

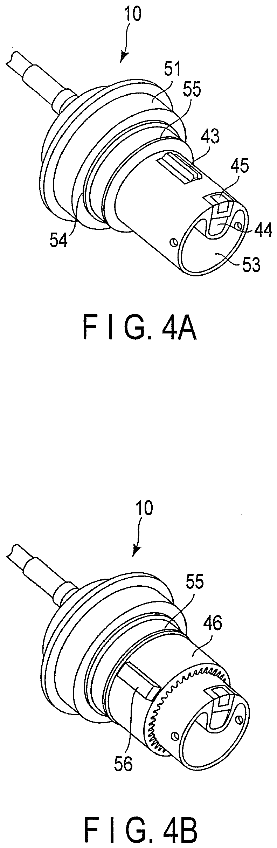

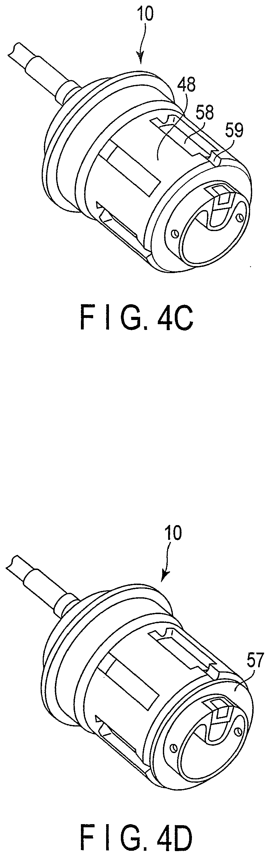

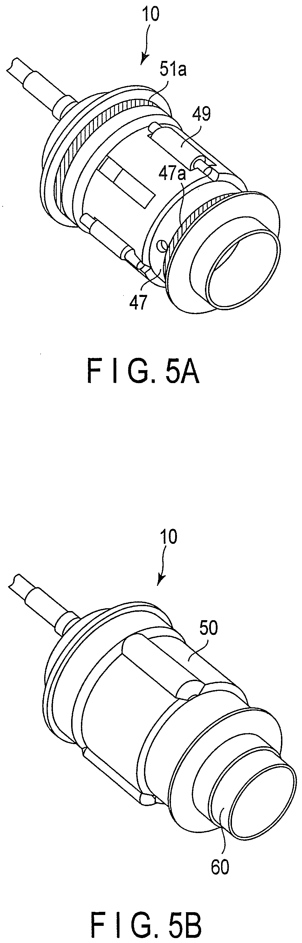

Next, with reference also to FIGS. 4A to 4D and FIGS. 5A to 5D, the structure of the rotational driving section 10 will be described in detail in accordance with an example of an assembly procedure. Here, FIGS. 4A to 4D are diagrams illustrating an assembly structure of the living body introduction apparatus. FIGS. 5A to 5D are diagrams continued from FIG. 4D and illustrating the assembly structure of the living body introduction apparatus. An outer rotating cylinder 8 of the rotating section 10 is configured separately from the spiral tube (the casing) 9, which is the introduction propulsion section.

In a conceptual sense, in the rotating section 10 of the living body introduction apparatus, the driving gear 41 coupled to the shaft 13 meshes with an inner gear 46 serving as a rotating member so that the driving gear 41 is disposed inside an inner rotating cylinder 48 via a transmission gear. The driving gear 41, the transmission gear 43, and the inner gear 46 provide a rotating mechanism. Rotation of the spiral tube 9 not only allows the rotation speed of the motor to be adjusted but also allows the rotation speed and torque to be adjusted by regulating the gear ratio of the above-described gears. The inner rotating cylinder 48 comprises a plurality of rollers rotatable installed on an outer circumferential surface of the inner rotating cylinder 48 and serving as a rotating site. The inner rotating cylinder 48 including the rollers is covered with a cover 50. Opening portions at the opposite ends of the tube-like cover 50 are fixedly bonded to a non-rotating site in a light-tight manner. Moreover, the outer rotating cylinder 8, comprising recesses formed therein and in which the rollers are housed, is fitted over the cover 50.

The living body introduction apparatus will be described below in detail.

As shown in FIG. 2, the driving gear 41 is housed in a gear cylinder 51. A shaft at the other end of the driving gear 41 is fitted in a hole in a retaining cylinder 44 fitted in the gear cylinder 51. The shaft is rotatable and is prevented from slipping out.

Moreover, the retaining cylinder 44 comprises a step formed therein by cutting out a part of an exposed outer circumferential surface side, and a through hole is formed in the step surface so as to extend in a radial direction. A retaining pin 45 is fitted in the through-hole, and a leading end of the pin is fixed in abutting contact with a recessed groove formed in the gear cylinder 51. An upper portion of the inserted retaining pin 45 lies in abutting contact with a rotating shaft of the transmission gear 43 to prevent the transmission gear 43 from slipping out.

As shown in FIG. 4A, an upper portion of the gear of the transmission gear 43 is located to be exposed through the cutout portion of the gear cylinder 51. As shown in FIG. 4B, the annular inner gear 46 is fitted over the gear cylinder 51. The inner gear 46 comprises a gear formed on an inner surface side of the inner gear 46 and meshing with the exposed transmission gear 43. Furthermore, an annular sliding plate 55 is fitted, in an intervening manner, over an inner step surface of the gear cylinder 51 which serves as a sliding surface allowing the gear cylinder 51 and the inner gear 46 to slide with respect to each other when the inner gear 46 is fitted into the gear cylinder 51. Similarly, an annular sliding plate 54 is fitted, in an intervening manner, over an outer step surface of the gear cylinder 51 which serves as a sliding surface allowing the gear cylinder 51 and the outer rotating cylinder 8 to slide with respect to each other when the outer rotating cylinder 8 is fitted over the gear cylinder 51.

Furthermore, the inner gear 46 comprises at least one rectangular key 56 formed in an outer circumferential surface of the inner gear 46. According to the present embodiment, by way of example, the single key 56 disposed on the outer circumferential surface is formed. This configuration allows the transmission gear 43 to rotate in conjunction with rotation of the driving gear 41, in turn rotating the gear of the inner gear 46. Thus, the inner gear 46 rotates on the gear cylinder 51.

Then, as shown in FIG. 4C, the inner rotating cylinder 48, in which three roller storage grooves 58 and a fitting key groove fitted over the key are formed, is fitted over the inner gear 46. In this case, the key 56 is fitted in the key groove.

The key 56 is formed such that a top portion of the key 56 lies as high as or slightly lower than an outer circumferential surface of the inner rotating cylinder 48. Thus, the outer circumferential surface including the top portion of the key 56 is formed to provide a generally smooth surface when the inner rotating cylinder 48 rotates. The rollers 49 are housed in the respective roller storage grooves 58. The rollers 49 are formed of hard metal such as stainless steel. Of course, the present embodiment is not limited to stainless steel, and any hard material is applicable.

As shown in FIG. 2 and FIG. 4C, each of the roller storage grooves 58 comprises two bearing grooves 59 formed at respective opposite ends in a longitudinal direction of the roller storage groove 58 to receive a roller shaft of the roller 49. The bearing grooves 59 are V- or U-shaped and the depth of each of the bearing grooves 59 is designed such that the roller surface of the roller 49 installed in the bearing groove 59 lies slightly away from a surface of the inner gear 46 and is thus free. When, for example, the diameter of the roller 49 is adjusted to synchronize rotation of the inner gear 46 with rotation of the roller, the surface of the inner gear 46 can be brought into contact with a surface of the roller 49 so that rotation of the roller itself helps the roller travel smoothly while passing the cover described below.

Then, as shown in FIG. 4D, the annular sliding plate 57 is fitted over the gear cylinder 51 so as to lie in front of the inner rotating cylinder 48. Moreover, as shown in FIG. 5A, the rollers 49 are installed in the respective roller storage grooves 58. The front cylinder 47 is fitted over the gear cylinder 51 so as to lie in front of the inner rotating cylinder 48, and fixed using screws or the like. The front cylinder 47 comprises a flange portion approximately in the center thereof, and a rear cylindrical portion of the front cylinder 47 is fitted over the gear cylinder 51.

In this configuration, the gear cylinder 51 and the front cylinder 47 are fixed to the gear cylinder 51, and only the inner rotating cylinder 48 located in the center is rotatable.

Then, as shown in FIG. 5B, the cover 50 covers the inner rotating cylinder 48 so that end portions of the cover contact an edge 51a of the gear cylinder 51 and an edge 47a of the front cylinder 47, respectively. As shown in FIG. 5A, the opposite end portions of the cover 50 are fixed in a liquid-tight manner to the edge 51a and the edge 47a, respectively. The liquid-tight fixation is performed using thread winding bonding, an adhesive, a double-sided adhesive tape, or the like.

The cover 50 is formed of a rubber material or a resin material that is non-conductive and stretchable, and prevents a liquid, vapor, or the like from permeating through the cover 50. The cover 50 is annularly shaped. For example, the cover 50 is shaped like a cylinder (a tube) of a size (a diameter) described below. Moreover, annular belts or the like may be fitted on the respective opposite ends of the cover 50 for fixation or reinforcement.

Further, a rear ferrule 60 of the front flexible hollow shaft 4a is fitted into a front cylinder portion of the flange portion of the front cylinder 47 and fixed using screws or the like. In this configuration, the opposite ends of the cover 50 are fixed to the gear cylinder 51 and the front cylinder 47. When the inner rotating cylinder 48, positioned in the center, rotates inside the cover 50, the rollers 49, installed in the inner rotating cylinder 48, rotate while pushing up the cover so that the cover 50 forms protruding portions.

A well-known adhesion inhibitor is sealed inside the cover 50 in order to prevent the abutting contact surfaces of the inner rotating cylinder 48 and the rollers 49 from adhering to an abutting contact surface of the cover 50 (blocking), thus maintaining slippage between the abutting contact surfaces. Moreover, to prevent the cover from being electrically charged (static electricity) in conjunction with rotation of the inner rotating cylinder 48, the members contacted by the cover 50 are desirably at ground potential.

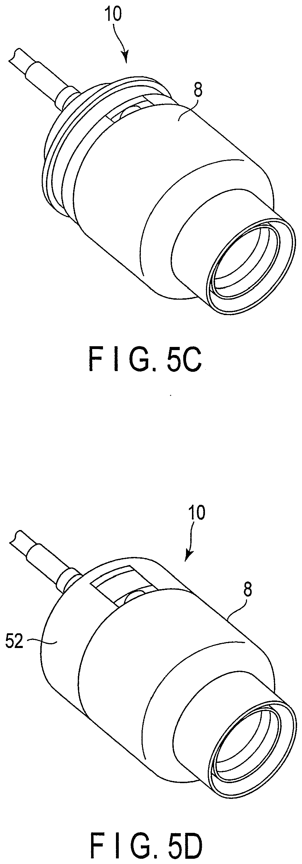

Then, as shown in FIG. 5C, the outer rotating cylinder 8 is fitted over the cover 50, that is, on an outer side of the inner rotating cylinder 48. As shown in section in FIG. 3A, the outer rotating cylinder 8 comprises circular grooves 8a formed at positions corresponding to the rollers 49 arranged in the inner rotating cylinder 48 and each of which has a size sufficient to contain the roller 49 covered with the cover 50. The circular groove 8a is adjusted to form a spacing between the outer rotating cylinder 8 and the cover 50 which serves to prevent the roller 49 covered with the cover 50. The roller 49 is always present, that is, always contained, in the circular groove 8a.

Rotation of the inner rotating cylinder 48 causes the protruding portions of the cover 50 formed by the rollers 49 to push the circular grooves 8a ahead to rotate the outer rotating cylinder 8. Furthermore, as shown in FIG. 5D, a retaining ring 52 is fitted over a base end side of the gear cylinder 51 so as to couple to the outer rotating cylinder 8. Here, the outer rotating cylinder 8 and the retaining ring 52 are coupled together using screws or the like. The surface of the coupled portion is covered with the installed spiral tube 9.

Now, the cover 50 will be described with reference to FIG. 2.

In the above-described pulling caused by slackening or creasing of the cover 50 with the opposite ends thereof fixed. As shown in FIG. 9A, when the cover 50 is subjected to a slack or crease 63, a leading end portion of the slack or crease comes into contact with the inner rotating cylinder and/or the outer rotating cylinder 8. Moreover, when the circular grooves 8a push the slack or crease ahead, the slack or crease moves as shown in FIG. 9B. In conjunction with this movement, a pulling area 64 may be formed in the cover 50 between the gear cylinder 51 and the front cylinder 47, thus damaging the cover 50; for example, tearing the cover 50. Thus, as shown in FIG. 2 and FIG. 5B, when the cover 50 covers the inner rotating cylinder 48 and the rollers 49, the cover 50 needs to contact the inner rotating cylinder 48 with no gap between the cover 50 and the inner rotating cylinder 48 and to be stretched so as to cover the rollers 49 with no gap between the cover 50 and the rollers 49.

Therefore, if the material of the cover 50 is not sufficiently stretchable, when the length of an outer circumference, shown in FIG. 3B, of the inner rotating cylinder 48 with the rollers 49 stored in the roller storage grooves 58 is denoted by an outer circumferential length C, and the length of inner circumference of the cover 50 before installation (covering) is denoted by an inner circumferential length D, the slack or crease 63 can be prevented by establishing a relation "outer circumferential length C ≥ inner circumferential length D". Here, FIG. 3B is a simplified diagram of the cross-sectional view in FIG. 3A showing that the rollers 49 are housed in the inner rotating cylinder 48. The length of a thick line in FIG. 3B is the outer circumferential length C. On the other hand, if the material of the cover 50 is stretchable, when the outer diameter of the inner rotating cylinder 48 is denoted by φA and the inner diameter of the cover 50 before installation (covering) is denoted by φB, the slack or crease 63 can be prevented by establishing a relation "outer circumferential length C > outer circumferential length of inner rotating cylinder 48 π x φA ≥ inner circumferential length of inner cover 50 π x φB = inner circumferential length D". The cover 50 stretched under these relations when covering the inner rotating cylinder 48 is in tight contact with the inner rotating cylinder 48. When the rollers 49 rotating on the outer circumferential surface of the inner rotating cylinder 48 come into abutting contact with the cover 50, movement of each of the rollers 49 causes a tensile force to be exerted in a portion positioned in a direction opposite to a rotating direction of the inner rotating cylinder 48 with respect to the roller 46. Thus, the tensile force stretches any slack generated by a push force by the roller 49 in a portion positioned in the rotating direction of the inner rotating cylinder 48, in the direction opposite to the rotating direction. As a result, the slack is prevented from being generated, and maintains the cover 50 in tight contact with the inner rotating cylinder 48.

Then, the protruding portions of the cover 50, formed by the rollers 49, push ahead the respective circular grooves 8a, formed inside the outer rotating cylinder 8, to rotate the outer rotating cylinder 8. The spiral tube 9 is fitted and installed on the outer rotating cylinder 8 to allow the spiral fin 7 with the propulsion function to rotate (forward). Rotating the spiral fin 7 backward (inverse rotation) allows a retraction function to be exerted.

Transmission of the rotational driving force of the whole living body introduction apparatus 6 according to the present embodiment will be described next.

The shaft 13 rotated by the motor 11 rotates the driving gear 41 and further rotates the inner gear 46 via the transmission gear 43, which meshes with the driving gear 41. First, the motor 11, functioning as a driving force generating section generating a rotational driving force rotating the inner gear 46, transmits the rotational driving force to the shaft 13, the driving gear 41, and the transmission gear 43, all of which function as a driving force transmitting section. The rotational driving force is transmitted through the shaft 13 from a base end thereof toward a leading end thereof where the driving gear 41 is provided. The inner rotating cylinder (inner rotor) 48 engages with the key 56, provided on the rotating inner gear 46, and rotates inside the cover 50 along with the inner gear 46. In other words, the inner rotating cylinder (inner rotor) 48 receives the rotational driving force transmitted by the shaft 13, the driving gear 41, and the transmission gear 43, all of which function as a driving force transmitting section, via the inner gear 46, provided on an inner circumferential portion of the inner rotating cylinder (inner rotor) 48 and functioning as a driving force receiving section. The inner rotating cylinder 48 then rotates around the longitudinal axis of the insertion section 2 with respect to the insertion section 2.

In this case, the rollers 49, provided on the outer circumferential surface of the inner rotating cylinder 48, form projecting portions (protruding portions) on the inner rotating cylinder 48. The rollers 49 come into abutting contact with the inner circumference of the cover 50 to push the cover 50 up toward the outside of the inner rotating cylinder 48, thus forming projecting portions (protruding portions) on the cover 50. The projecting portions (protruding portions) formed by the rollers 49 and the cover 50 are fitted into the circular grooves 8a, formed in the outer rotating cylinder (outer rotor) 8, and push an inner wall surface forming circular grooves 61a ahead in conjunction with rotation of the inner rotating cylinder 48. In other words, the outer rotating cylinder 8 comprises engagement sections provided on the inner wall surface of the outer rotating cylinder 8 and engaged by the projecting portions formed by the rollers 49 and the cover 50. The engagement sections are pushed ahead to rotate the outer rotating cylinder 8 along with the inner rotating cylinder 48. Thus, the spiral tube 9 fitted on the outer rotating cylinder 8 is rotated.

As described above, the present embodiment provides a highly resistant liquid-tight structure with a simple configuration and allows the spiral fin disposed on the insertion section to rotate.

Since the cover 50 is equal to or smaller than the inner rotating cylinder 48 in size, the cover 50 avoids rising from the inner rotating cylinder 48 and the rollers 49. This enables prevention of pulling between a fixed area and both the inner rotating cylinder 48 and the outer rotating cylinder 8 and thus prevention of possible damage to the cover, for example, tearing of the cover and the cover peeling off from the fixed area. As a result, a liquid-tight state can be maintained.

Furthermore, the present embodiment provides a configuration in which the rollers 49 push the circular grooves 8a in the outer rotating cylinder 8 ahead via the cover 50 to rotate the outer rotating cylinder 8. The configuration eliminates the need for such small rollers as involved in the conventional technique and is thus simple. This is also effective for reducing costs. Furthermore, omission of structural members further improves the effect of sterilization.

Since the outer rotating cylinder 8 is separate from the spiral tube 9, the spiral tube 9 can be selectively installed which comprises a fin suitable for a lumen to be observed. Here, when an inner circumferential surface of the spiral tube 9 and an outer circumferential surface of the outer rotating cylinder 8 are shaped like a polygon such as an octagon and formed to be engageable with each other, the spiral tube 9 with the insertion section 2 inserted along a longitudinal axis of the spiral tube 9 can be fixed to the outer circumferential surface of the outer rotating cylinder 8. Furthermore, replacement only of the spiral tube 9 that is expandable allows a reduction in costs.

### [Second Embodiment]

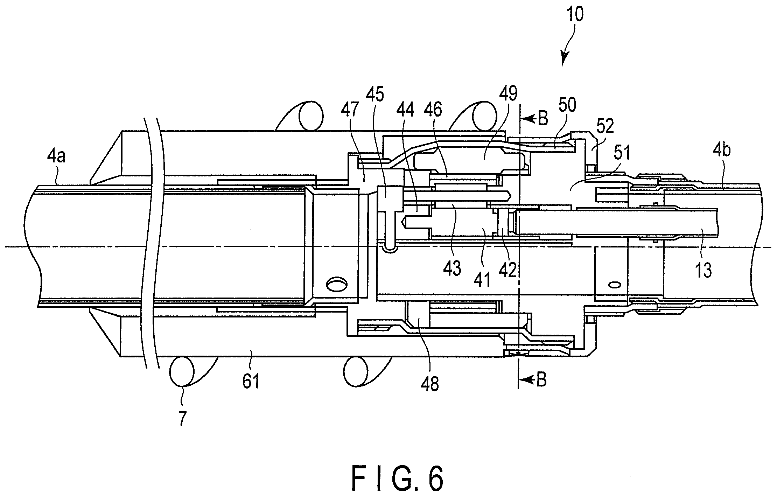

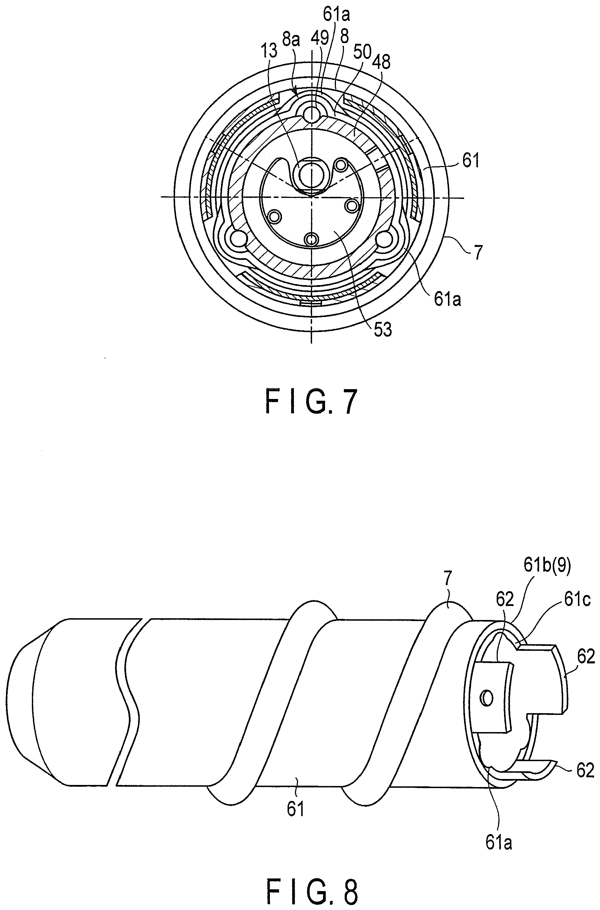

FIG. 6 is a diagram showing a configuration of a living body introduction apparatus according to a second embodiment in section. FIG. 7 is a cross-sectional view showing the configuration and taken along B-B in FIG. 6. FIG. 8 is a diagram showing an external configuration of an outer rotating cylinder configured integrally with a spiral tube (a casing) of the living body introduction apparatus. Structural sites of the present embodiment which are equivalent to corresponding structural sites of the first embodiment are denoted by the same reference numerals.

In an endoscope apparatus 1 according to the present embodiment, a manipulation section and a driving unit system are configured equivalently to the manipulation section and driving unit system according to the above-described first embodiment, respectively. The present embodiment differs from the first embodiment in that a spiral tube 9 and an outer rotating cylinder 8 of a living body introduction apparatus 6 on an insertion section 2 are integrated together. Here, the description of the manipulation section and the driving unit system is omitted, and the living body introduction apparatus 6 will be described.

In a conceptual sense, in the living body introduction apparatus 6 according to the present embodiment, a driving gear 41 coupled to a shaft 13 meshes, via a transmission gear 43, with an inner gear 46 disposed inside an inner rotating cylinder 48. A plurality of rollers are rotatably installed on the inner rotating cylinder 48. The inner rotating cylinder 48 including the rollers is covered with a cover 50. Opposite ends of the cover 50 are fixedly bonded to non-rotating sites in a liquid-tight manner. The above-described configuration is equivalent to the corresponding configuration of the above-described first embodiment.

As is the case with the first embodiment, the cover 50 covers the inner rotating cylinder so that opposite end portions of the cover contact a gear cylinder 51 and a front cylinder 47. The cover 50 is fixed in a liquid-tight manner using thread winding bonding, an adhesive, a double-sided adhesive tape, or the like.

Thus, if the material of the cover 50 is not sufficiently stretchable, when the length of an outer circumference of the inner rotating cylinder 48 with rollers 49 stored in roller storage grooves 58 is denoted by an outer circumferential length C as shown in FIG. 3B, and the length of inner circumference of the cover 50 before installation (covering) is denoted by an inner circumferential length D, a possible slack or crease 63 can be prevented by establishing a relation "outer circumferential length C ≥ inner circumferential length D". On the other hand, if the material of the cover 50 is stretchable, when the outer diameter of the inner rotating cylinder 48 is denoted by φA and the inner diameter of the cover 50 before installation (covering) is denoted by φB, the slack or crease 63 can be prevented by establishing a relation "outer circumferential length C > outer circumferential length of inner rotating cylinder 48 π x φA ≥ inner circumferential length of inner cover 50 π x φB = inner circumferential length D".

An outer rotating cylinder 61 according to the present embodiment is configured such that a spiral tube with a spiral fin formed thereon is integrally mounted on an outer circumferential surface of the outer rotating cylinder 61. The outer rotating cylinder 61 is fitted over the cover 50.

As shown in FIG. 7, a base end-side opening portion of the outer rotating cylinder 61 fitted over the inner rotating cylinder 48 including the rollers has a two-layer structure with a soft spiral tube portion 61b and a metal portion or hard resin portion (the portion corresponding to the outer rotating cylinder 8 according to the first embodiment) 61C. Recesses are formed on an inner surface side of the hard portion so as to house the rollers 49. Each of the recesses is shaped like a circle, a rectangle, or the like so to be able to house the roller 49.

Furthermore, as shown in FIG. 8, the outer rotating cylinder 61 comprises a plurality of mounting sections 62 provided at a base end-side opening end of the hard portion and extending in a longitudinal direction so as to couple to a retaining ring 52. The mounting sections 62 and the retaining ring 52 are coupled together using screws or the like. Coupling portions including the screws are covered with insulating partial covers (not shown in the figures). The outer rotating cylinder 61 is fitted over the inner rotating cylinder 48 via the cover 50 so that the rollers 49 covered with the cover 50 are contained in the recesses as shown in FIG. 6 and FIG. 7. Each of the recesses is shaped like a circle, a rectangle, or the like so to be able to house the roller 49. A case where circular grooves 61a are formed as the recesses will be described below.

In the living body introduction apparatus 6 according to the preset embodiment, the shaft 13 rotated by a motor 11 rotates the inner gear 46 via the transmission gear 43, which meshes with and rotates the driving gear 41. First, the motor 11, functioning as a driving force generating section generating a rotational driving force rotating the inner gear 46, transmits the rotational driving force to the shaft 13, the driving gear 41, and the transmission gear 43, all of which function as a driving force transmitting section. The rotational driving force is transmitted through the shaft 13 from a base end thereof toward a leading end thereof where the driving gear 41 is provided. The rotational driving force is then transmitted to the inner gear 46 via the driving gear 41 and the transmission gear 43. The inner rotating cylinder (inner rotor) 48 engages with a key 56 provided on the rotating inner gear 46 and rotates inside the ?rubber (cover) ? 50 along with the inner gear 46. In other words, the inner rotating cylinder (inner rotor) 48 receives the rotational driving force transmitted by the shaft 13, the driving gear 41, and the transmission gear 43, all of which function as a driving force transmitting section, via the inner gear 46, provided on an inner circumferential portion of the inner rotating cylinder (inner rotor) 48 and functioning as a driving force receiving section. The inner rotating cylinder 48 then rotates around a longitudinal axis of the insertion section 2 with respect to the insertion section 2.

In this case, the rollers 49, provided on the outer circumferential surface of the inner rotating cylinder 48, form projecting portions (protruding portions) on the inner rotating cylinder 48. The rollers 49 come into abutting contact with the inner circumference of the cover 50 to push the cover 50 up toward the outside of the inner rotating cylinder 48, thus forming projecting portions (protruding portions) on the cover 50. The projecting portions (protruding portions) formed by the rollers 49 and the cover 50 are fitted into the circular grooves 61a, formed in the outer rotating cylinder 61 (outer rotor), and push an inner wall surface forming the circular grooves 61a ahead in conjunction with rotation of the inner rotating cylinder 48. In other words, the outer rotating cylinder 61 (outer rotor) comprises engagement sections provided on the inner wall surface of the outer rotating cylinder 61 (outer rotor) and engaged by the projecting portions formed by the rollers 49 and the cover 50. The engagement sections are pushed ahead to rotate the outer rotating cylinder 61 (outer rotor) along with the inner rotating cylinder 48.

As described above, the present embodiment is effective for reducing costs using a simple configuration, provides a highly resistant liquid-tight structure, and allows the spiral fin disposed on the insertion section to rotate. Since the cover 50 is equal to or smaller than the inner rotating cylinder 48 in size as is the case with the above-described first embodiment, the cover can be prevented from being damaged, for example, being torn and from being peeled off from a fixed area. As a result, a liquid-tight state can be maintained. Furthermore, the outer rotating cylinder integrated with the spiral tube is formed to be inserted through the insertion section 2 along the longitudinal axis of the insertion section 2. The outer rotating cylinder is further formed such that the projecting portions (protruding portions) formed by the cover 50 and the rollers 49 are removably fitted into the recesses (circular grooves or engagement sections) in the outer rotating cylinder. Thus, the outer rotating cylinder can be easily installed and removed.

## Claims

1. An introduction apparatus (6) comprising:
an insertion section (2) configured to extend along a longitudinal axis and to be able to be inserted into a lumen;
a manipulation section (3) disposed on a base end side of the insertion section (2) and configured in such a manner that a rotational driving source is housed in the manipulation section (3) which generates rotational driving force;
an inner rotating cylinder (48) provided in the insertion section (2) at any position on the insertion section (2) and annularly configured to be rotatable around the longitudinal axis, the inner rotating cylinder (48) having a plurality of rollers (49) rotatably installed on an outer circumferential surface of the inner rotating cylinder (48) and rotating in accordance with the rotational driving force from the rotational driving source; a covering member (50) that is stretchable and fixed at opposite ends thereof to non-rotating sites located beyond the inner rotating cylinder (48),
an outer rotating cylinder (8, 61) comprising an engagement section including a plurality of circular grooves (8a, 61a) formed at positions corresponding to the plurality of rollers (49) arranged in the inner rotating cylinder (48), each of the circular grooves (8a, 61a) having a size sufficient to contain a respective roller (49) covered with the covering member (50), the outer rotating cylinder (8, 61) being fitted on a casing side of the inner rotating cylinder (48) covered with the covering member (50), such that the rotation of the inner rotating cylinder (48) causes the protruding portions of the cover (50) formed by the plurality of rollers (49) to push the circular grooves (8a, 61a) ahead to rotate the outer rotating cylinder (8, 61); and
an introduction propulsion unit rotatable along with the outer rotating cylinder (8, 61) and comprising a spiral propulsion site on an outer surface of the introduction propulsion unit, the introduction propulsion unit supporting rotational introduction into the lumen;
**characterized in that**
the covering member (50) is configured to have an inner circumferential length equal to or smaller than an outer circumferential length of the inner rotating cylinder (48) including the plurality of rollers (49), the covering member (50) annularly covering the inner rotating cylinder (48) including the plurality of rollers (49) in tight contact with the inner rotating cylinder (48) when assembled in a stretched manner,
the covering member (50), when covering the inner rotating cylinder (48) and the plurality of rollers (49), further configured to contact the inner rotating cylinder (48) with no gap between the cover (50) and the inner rotating cylinder (48), and to be stretched so as to cover the plurality of rollers (49) with no gap between the covering member (50) and the plurality of rollers (49).

2. The introduction apparatus (6) according to claim 1, **characterized in that** the plurality of rollers (49) are disposed rotationally symmetrically with respect to the longitudinal axis and configured to move rotationally on the covering member (50) between the rotating inner rotating cylinder (48) and the fixed covering member (50).

3. The introduction apparatus (6) according to claim 1, wherein the propulsion site is spirally formed along the longitudinal axis of the outer rotating cylinder (8, 61).

4. The introduction apparatus (6) according to claim 1, **characterized in that** the insertion section (2) comprises a bending section (5) at a position closer to a leading end than the introduction propulsion unit.

5. The introduction apparatus (6)according to claim 1, comprising a flexible shaft (4a, 4b) configured to couple the rotational driving source to the inner rotating cylinder (48) through the insertion section (2) to transmit a rotating force.

6. The introduction apparatus (6) according to claim 5, **characterized by** comprising a rotating mechanism disposed between the inner rotating cylinder (48) and the shaft (4a, 4b) and comprising a plurality of gears configured to convert rotation from the rotational driving source into any rotation speed and a rotating force.

## Patentansprüche

1. Einführungsvorrichtung (6), umfassend:
einen Einführabschnitt (2), der dazu ausgelegt ist, sich entlang einer Längsachse zu erstrecken und in ein Lumen eingeführt werden zu können;
einen Handhabungsabschnitt (3), der auf einer Basisendseite des Einführabschnitts (2) angeordnet und derart ausgelegt ist, dass eine Drehantriebsquelle in dem Handhabungsabschnitt (3) untergebracht ist, die eine Drehantriebskraft erzeugt;
einen inneren Drehzylinder (48), der in dem Einführabschnitt (2) an einer beliebigen Position an dem Einführabschnitt (2) vorgesehen und ringförmig ausgelegt ist, um rund um die Längsachse gedreht werden zu können, wobei der innere Drehzylinder (48) eine Mehrzahl Rollen (49) aufweist, die drehbar an einer Außenumfangsfläche des inneren Drehzylinders (48) angebaut sind und sich gemäß der Drehantriebskraft aus der Drehantriebsquelle drehen;
ein Abdeckelement (50), das dehnbar und an entgegengesetzten Enden davon an drehungsfreien Stellen, die sich außerhalb des inneren Drehzylinders (48) befinden, fixiert ist,
einen äußeren Drehzylinder (8, 61), der einen Einrückabschnitt umfasst, welcher eine Mehrzahl kreisförmiger Nuten (8a, 61a) umfasst, die an Positionen gebildet sind, die der in dem inneren Drehzylinder (48) angeordneten Mehrzahl Rollen entsprechen, wobei jede der kreisförmigen Nuten (8a, 61a) eine Größe aufweist, die ausreicht, um eine entsprechende mit dem Abdeckelement (50) abgedeckte Rolle (49) zu enthalten, wobei der äußere Drehzylinder (8, 61) an einer Gehäuseseite des inneren Drehzylinders (48) angebracht ist, der mit dem Abdeckelement (50) abgedeckt ist, sodass die Drehung des inneren Drehzylinders (48) bewirkt, dass die vorragenden Abschnitte der Abdeckung (50), die durch die Mehrzahl Rollen (49) gebildet sind, die kreisförmigen Nuten (8a, 61a) vorwärtstreiben, um den äußeren Drehzylinder (8, 61) zu drehen; und
eine Einführungsantriebseinheit, die zusammen mit dem äußeren Drehzylinder (8, 61) gedreht werden kann und eine spiralförmige Antriebsstelle an einer Außenfläche der Einführungsantriebseinheit umfasst, wobei die Einführungsantriebseinheit die Dreheinführung in das Lumen unterstützt;
**dadurch gekennzeichnet, dass**
das Abdeckelement (50) dazu ausgelegt ist, eine Innenumfangslänge aufzuweisen, die gleich oder kleiner ist als eine Außenumfangslänge des die Mehrzahl Rollen (49) umfassenden inneren Drehzylinders (48), wobei das Abdeckelement (50) den die Mehrzahl Rollen (49) umfassenden inneren Drehzylinder (48) in engem Kontakt mit dem inneren Drehzylinder (48) ringförmig abdeckt, wenn in gedehnter Weise montiert,
das Abdeckelement (50), wenn es den inneren Drehzylinder (48) und die Mehrzahl Rollen (49) abdeckt, ferner dazu ausgelegt ist, den inneren Drehzylinder (48) zu kontaktieren, ohne Lücke zwischen der Abdeckung (50) und dem inneren Drehzylinder (48), und derart gedehnt zu werden, dass es die Mehrzahl Rollen (49) ohne Lücke zwischen dem Abdeckelement (50) und der Mehrzahl Rollen (49) abdeckt.

2. Einführungsvorrichtung (6) nach Anspruch 1, **dadurch gekennzeichnet, dass** die Mehrzahl Rollen (49) rotationssymmetrisch mit Bezug auf die Längsachse angeordnet und dazu ausgelegt sind, sich an dem Abdeckelement (50) zwischen dem rotierenden inneren Drehzylinder (48) und dem fixierten Abdeckelement (50) drehend zu bewegen.

3. Einführungsvorrichtung (6) nach Anspruch 1, wobei die Antriebsstelle spiralförmig entlang der Längsachse des äußeren Drehzylinders (8, 61) gebildet ist.

4. Einführungsvorrichtung (6) nach Anspruch 1, **dadurch gekennzeichnet, dass** der Einführungsabschnitt (2) einen Biegeabschnitt (5) in einer Position umfasst, die näher an einem vorderen Ende als an der Einführungsantriebseinheit liegt.

5. Einführungsvorrichtung (6) nach Anspruch 1, umfassend eine flexible Welle (4a, 4b), die dazu ausgelegt ist, die Drehantriebsquelle mit dem inneren Drehzylinder (48) durch den Einführungsabschnitt (2) zu koppeln, um eine Drehkraft zu übertragen.

6. Einführungsvorrichtung (6) nach Anspruch 5,
**gekennzeichnet durch**:
Umfassen eines Drehmechanismus, der zwischen dem inneren Drehzylinder (48) und der Welle (4a, 4b) angeordnet ist, und Umfassen einer Mehrzahl Zahnräder, die dazu ausgelegt sind, die Drehung von der Drehantriebsquelle in eine beliebige Drehgeschwindigkeit und eine Drehkraft umzuwandeln.

## Revendications

1. Appareil d'introduction (6) comprenant :
une section d'insertion (2) configurée pour s'étendre le long d'un axe longitudinal et pour être apte à être insérée dans une lumière ;
une section de manipulation (3) disposée sur un côté extrémité de base de la section d'insertion (2) et configurée de telle sorte qu'une source d'entraînement en rotation est logée dans la section de manipulation (3), laquelle génère une force d'entraînement en rotation ;
un cylindre rotatif interne (48) disposé dans la section d'insertion (2) dans une position quelconque sur la section d'insertion (2) et configuré de manière annulaire pour être apte à tourner autour de l'axe longitudinal, le cylindre rotatif interne (48) ayant une pluralité de rouleaux (49) installés de manière rotative sur une surface circonférentielle externe du cylindre rotatif interne (48) et tournant conformément à la force d'entraînement en rotation provenant de la source d'entraînement en rotation ;
un élément de recouvrement (50) qui est étirable et fixé, au niveau d'extrémités opposées de celui-ci, à des emplacements non-rotatifs situés au-delà du cylindre rotatif interne (48),
un cylindre rotatif externe (8, 61) comprenant une section d'engagement comprenant une pluralité de rainures circulaires (8a, 61a) formées à des positions correspondant à la pluralité de rouleaux (49) disposés dans le cylindre rotatif interne (48), chacune des rainures circulaires (8a, 61a) ayant une taille suffisante pour contenir un rouleau respectif (49) recouvert de l'élément de recouvrement (50), le cylindre rotatif externe (8, 61) étant monté sur un côté boîtier du cylindre rotatif interne (48) recouvert de l'élément de recouvrement (50), de telle sorte que la rotation du cylindre rotatif interne (48) amène les parties en saillie du recouvrement (50) formées par la pluralité de rouleaux (49) à pousser les rainures circulaires (8a, 61a) vers l'avant pour faire tourner le cylindre rotatif externe (8, 61) ; et
une unité de propulsion d'introduction apte à tourner conjointement avec le cylindre rotatif externe (8, 61) et comprenant un emplacement de propulsion en spirale sur une surface externe de l'unité de propulsion d'introduction, l'unité de propulsion d'introduction supportant une introduction rotative dans la lumière ;
**caractérisé par le fait que**
l'élément de recouvrement (50) est configuré pour avoir une longueur circonférentielle interne égale ou inférieure à une longueur circonférentielle externe du cylindre rotatif interne (48) comprenant la pluralité de rouleaux (49), l'élément de recouvrement (50) recouvrant de manière annulaire le cylindre rotatif interne (48) comprenant la pluralité de rouleaux (49) en contact étroit avec le cylindre rotatif interne (48) lorsqu'assemblé de manière étirée,
l'élément de recouvrement (50), lorsqu'il recouvre le cylindre rotatif interne (48) et la pluralité de rouleaux (49), étant en outre configuré pour entrer en contact avec le cylindre rotatif interne (48) sans intervalle entre le recouvrement (50) et le cylindre rotatif interne (48), et pour être étiré de façon à recouvrir la pluralité de rouleaux (49) sans intervalle entre l'élément de recouvrement (50) et la pluralité de rouleaux (49).

2. Appareil d'introduction (6) selon la revendication 1, **caractérisé par le fait que** la pluralité de rouleaux (49) sont disposés de manière symétrique et rotative par rapport à l'axe longitudinal et configurés pour se déplacer de manière rotative sur l'élément de recouvrement (50) entre le cylindre rotatif interne (48) en rotation et l'élément de recouvrement (50) fixe.

3. Appareil d'introduction (6) selon la revendication 1, **caractérisé par le fait que** l'emplacement de propulsion est formé en spirale le long de l'axe longitudinal du cylindre rotatif externe (8, 61).

4. Appareil d'introduction (6) selon la revendication 1, **caractérisé par le fait que** la section d'insertion (2) comprend une section de courbure (5) à une position plus proche d'une extrémité avant que l'unité de propulsion d'introduction.

5. Appareil d'introduction (6) selon la revendication 1, comprenant un arbre souple (4a, 4b) configuré pour accoupler la source d'entraînement en rotation au cylindre rotatif interne (48) par l'intermédiaire de la section d'insertion (2) pour transmettre une force de rotation.

6. Appareil d'introduction (6) selon la revendication 5, **caractérisé par le fait qu'**il comprend un mécanisme de rotation disposé entre le cylindre rotatif interne (48) et l'arbre (4a, 4b) et comprenant une pluralité d'engrenages configurés pour convertir une rotation à partir de la source d'entraînement en rotation en n'importe quelle vitesse de rotation et une force de rotation.
